# EUROPEAN PATENT SPECIFICATION

(11) **EP 1 269 942 B1**
(45) Date of publication and mention of the grant of the patent: **01.06.2005**
(21) Application number: 02013891.3
(22) Date of filing: 24.06.2002
(51) Int. Cl.: A61F 5/02

(54) **Physiotherapy brace**
Orthopädische Stütze
Support orthopédique

(30) Priority: 25.06.2001 SM 200100013
(43) Date of publication of application: 02.01.2003
(73) Proprietor: Coema S.r.l., 47891 Rovereta-Falciano (SM)
(72) Inventor: Cardella, Giancarlo, Colbordolo (PV) (IT)
(74) Representative: Provvisionato, Paolo

(56) References cited:
- CH-A- 678 147
- GB-A- 686 073
- US-A- 3 274 996
- US-A- 5 370 133
- US-A- 5 584 072

## Description

The present invention relates to a physiotherapy brace.

Both in post-operative therapy and during physiotherapy treatment generally for the dorsal and lumbar region the use of braces instead of plaster jackets has become more widespread during recent years. Without restricting the general nature of their use, by way of example it must be mentioned that they have been used successfully both for traumatic or pathological fractures and in the case of vertebral osteoporosis and osteomalacia involving fractures.

The braces currently used comprise, as described in patents CH 678 147, US-A-3 274 996 and GB 686 073 A, a metal frame, with belt, which supports the padded parts located in the areas where contact with the patient's body occurs. The padded parts generally rest, as well as on the sides, at the front on the chest and on the lower stomach and at the rear on the central zone of the back; also they may be fixed or movable to a small degree so as to be able to adapt to the anatomical configuration of the patient, without however limiting functioning of the brace. Since, however, it is required to keep the patient's trunk adequately rigid, the brace is arranged in place by means of a system for tensioning and tightening the belt, which may be used in various ways, with specific characteristics, advantages and drawbacks. A first solution envisages the use of a lever-type metal closure which is movable in a front-to-rear direction during closing and vice versa during opening; in a more recent solution, however, a rotational closing system made with plastic materials is used.

The main drawback encountered in these instruments is precisely associated with the closing system: the tension of the belt and tightening in fact require an effort such that the patient has difficulty in performing adjustment alone, in particular because locking of the closing system involves movement thereof when the belt is already tensioned. Moreover, the first system is not safe because even an involuntary movement of the upper limbs may result in accidental opening thereof, while the second system requires the patient to effect a rotational movement in the side region which is difficult to perform.

In the most recent brace, forming the subject of patent No. RN2000A000030 dated June 2000, the belt also has drawbacks: it is made of a rigid plastic material which, although it allows simple adjustment, results in a projecting surplus part which is bothersome for the patient and in fact obliges the therapist to remove the part in question.

Apart from the inconvenience per se, the instruments available to the person performing cutting are not always suitable for cutting such a material with the result that the remaining part may have an irregular edge which is sharp and generally dangerous for the patient.

The object of the present invention is therefore that of eliminating the drawbacks mentioned above.

The invention, as characterized by the claims, achieves the object using a closing system with a rotating element, locking of which is performed automatically at the end of a simple front-to-rear or rear-to-front rotational movement thereof.

The main advantage obtained by means of the present invention consists essentially in the fact that the patient manages to tension the belt and tighten the closure with a limited amount of effort, without the risk of involuntarily releasing the closing system with an accidental movement of the upper limbs. Moreover, any removal of material is avoided, while maintaining the possibility of adjusting the width of the belt. Finally, an identical rotating element can be mounted on both sides of the brace so as to be adaptable to the use of both right-handed and left-handed patients.

Further advantages and characteristic features of the invention will emerge more clearly from the detailed description which follows, with reference to the accompanying drawings, which show a non-limiting example of embodiment thereof in which:

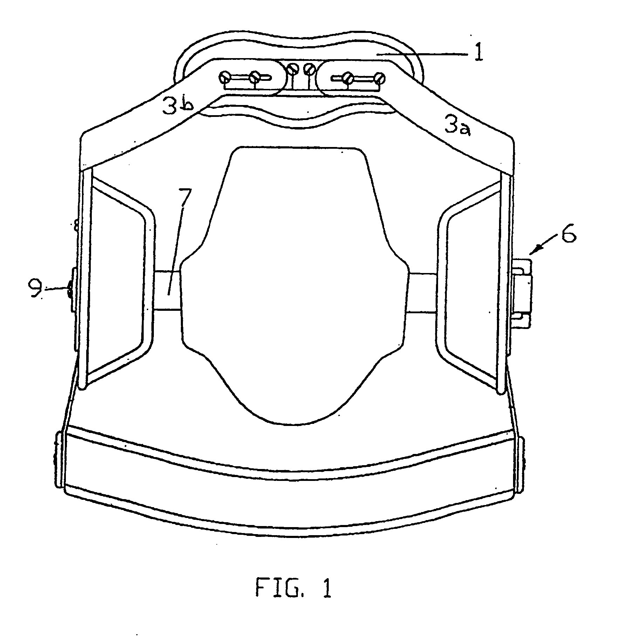

- Figure 1 shows a front view of the invention;

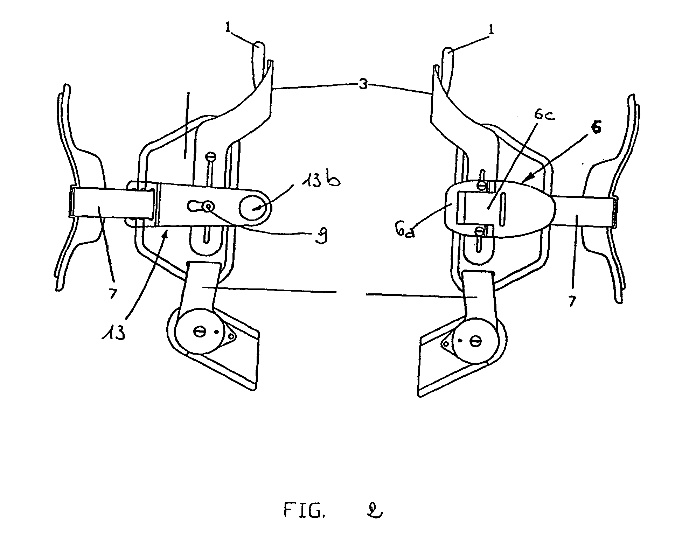

- Figure 2 show two side views of the invention;

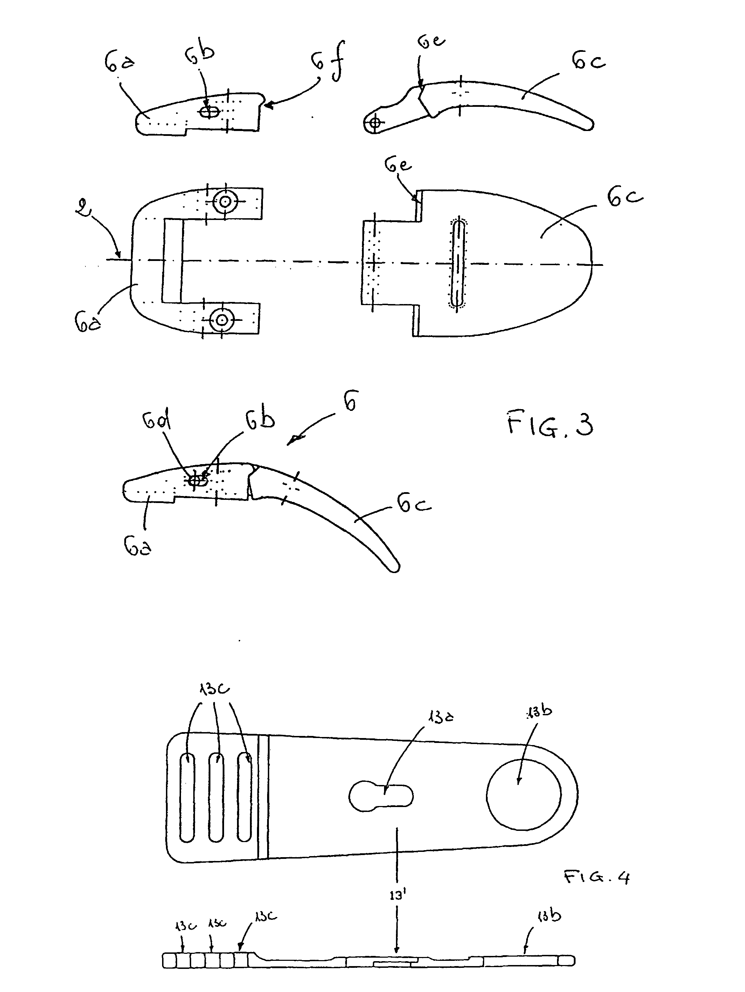

- Figure 3 shows a side and plan view of a detail of the invention, prior to assembly, and a side view after assembly;
- Figure 4 shows a side view and plan view of a second detail of the invention.

As can be seen from the figures, the invention relates to a physiotherapy brace comprising a metal frame (1) which is partly padded and a belt which joins one side (3a) thereof to the other side (3b), fixing of the belt (7) being performed by means of a snap-engagement closing device (6) able to lock automatically at the end of a front-to-rear or rear-to-front rotational movement thereof.

The closing device (6) comprises a fixed element (6a) which is integrally joined to one side (3a) and a movable element (6c) which is fastened to the belt (7) and rotates with respect to a pin (6d) to which it is integrally joined. In turn, the pin (6d) slides inside an eyelet (6b) formed on the surface of the fixed element (6a), while the movable element (6c) passes from the opening position to the closing position, tensioning the belt (7) and engaging by means of its recessed part (6e) with a corresponding projection (6f) of the fixed element (6a). The displacement of the pin (6d) inside the eyelet (6b) allows the movable element (6c) to pass easily over the projection (6f) of the fixed element (6a) during closing, while tensioning of the belt (7) provides a resistance sufficient to prevent involuntary opening of the closing device (6) which can be achieved only with a certain effort on the part of the patient, performing a backwards rotation of the movable element (6c).

The belt (7) supports means (13) for stable, but removable association with the metal frame (1) so as to allow fastening to one side (3b) prior to tensioning and locking of the closing device (6). Said means consist of a shaped buckle (13') provided with an eyelet (13a) formed in a manner complementing a fixed pin (9) 'of the metal frame (1), so that the head of the pin (9) can be inserted into the eyelet (13a) but can no longer be accidentally released therefrom after the belt (7) has been tensioned. In order to facilitate these operations, the buckle (13') comprises a grip (13b) which can be easily operated by the patient and is able to tension the belt (7) so as to cause the association means (13) to interfere with the pin (9) of the metal frame (1), when the brace is worn, and disengage them, when the brace must be removed.

The buckle (13') also comprises at least one through-slit (13c) - three in number in the example shown in the figures - for modifying the tension of the belt (7) and making it adjustable to fit the patient.

Finally, the closing device (6) comprises all the elements perfectly symmetrical with respect to a transverse axis and therefore also has an operational symmetry with respect thereto. This allows advantageously the same closing device (6) to be used on both sides (3a, 3b) of the brace, therefore allowing its use to be adapted to both right-handed and left-handed patients without the need to manufacture further moulds.

The invention thus conceived may be subject to numerous modifications and variations, all falling within the scope of the inventive idea. Moreover, all the details may be replaced by technically equivalent elements.

In practice obviously modifications and/or improvements are possible, nevertheless within the scope of the following claims.

## Claims

1. Physiotherapy brace comprising a metal frame (1) which is partly padded and a belt (7) which joins one side (3a) thereof to the other side (3b), comprising a snap-engagement closing device (6) which is able to lock automatically at the end of a front-to-rear or rear-to-front rotational movement thereof, the closing device (6) comprising a fixed element (6a) which is integrally joined to one side (3a) and a movable element (6c) which is fastened to the belt (7) and rotates with respect to a pin (6d) to which it is integrally joined, said pin (6d) being sliding inside an eyelet (6b) formed on the surface of the fixed element (6a), while the movable element (6c) passes from the opening position to the closing position, tensioning the belt (7) and engaging by means of its recessed part (6e) with a corresponding projection (6f) of the fixed element (6a), **characterised in that** said pin (6d) is displaceably sliding inside said eyelet (6b).

2. Brace according to Claim 1, **characterized in that** the belt (7) supports means (13) for stable, but removable association with the metal frame (1).

3. Brace according to Claim 2, **characterized in that** the association means (13) consist of a shaped buckle (13').

4. Brace according to Claim 3, **characterized in that** the buckle (13') comprises an eyelet (13a) shaped in a manner complementing a pin (9) of the metal frame (1).

5. Brace according to Claim 4, **characterized in that** the buckle (13') comprises a grip (13b) able to tension the belt so as to engage and disengage the eyelet (13a) and the pin (9).

6. Brace according to Claim 3, **characterized in that** the buckle (13') comprises at least one through-slit (13c) for modifying the tension of the belt (7).

7. Brace according to one of the preceding claims, **characterized in that** the closing device (6) is symmetrical both structurally and functionally with respect to an axis (2) so that it can be used equally well on both the sides (3a, 3b).

## Patentansprüche

1. Orthopädische Stütze, umfassend einen teilweise ausgepolsterten Metallrahmen (1) und einen Riemen (7), der eine Flanke (3a) mit der anderen Flanke (3b) vereint, umfassend eine Schnappschließvorrichtung (6), die am Ende eines eigenen Ausschlages nach vornehinten oder hinten-vorne sich selbsttätig festklemmt, wobei die Schnappschließvorrichtung (6) mindesten ein an einer Flanke (3a) festliegendes Element (6a) und ein bewegliches, am Riemen (7) angesetztes Element (6c) umfasst, das gegenüber einem Bolzen (6d) schwenkbar ist, an dem es festliegt, wobei der Bolzen (6d) innerhalb eines in der Oberfläche des festen Elementes (6a) ausgenommenen Langloches (6b) beweglich ist, während das bewegliche Element (6c) von der Öffnungsstellung in die Schließstellung übergeht, indem der Riemen (6) gespannt und einen eigenen Durchbruch (6e) an einer entsprechenden Nase (6f) des festen Elementes (6a) in Eingriff bringt, **dadurch gekennzeichnet, dass** der Bolzen (6d) innerhalb des Langloches (6b) verschiebbar ist.

2. Stütze nach Anspruch 1 oder 2, **dadurch gekennzeichnet, dass** der Riemen (7)stabile, jedoch abnehmbare Mittel (13) trägt, die dem Metallrahmen (1) zugeordnet sind.

3. Stütze nach Anspruch 2, **dadurch gekennzeichnet, dass** die zugeordneten Mittel (13) aus einer profilierten Schnalle (13') bestehen.

4. Stütze nach Anspruch 3, **dadurch gekennzeichnet, dass** die Schnalle (13') ein Langloch (13a) aufweist, das zum Bolzen (9) des Metallrahmens (1) komplementär profiliert ist.

5. Stütze nach Anspruch 4, **dadurch gekennzeichnet, dass** die Schnalle (13') einen Griff (13b) umfasst, der den Riemen (7) derart spannt, dass das Langloch (13a) und der Bolzen (9) in und außer Eingriff gebracht werden.

6. Stütze nach Anspruch 3, **dadurch gekennzeichnet, dass** die Schnalle (13') mindestens einen Durchlauf (13c) umfasst, um die Spannung des Riemens (7) zu ändern.

7. Stütze nach einem der voranstehenden Ansprüche, **dadurch gekennzeichnet, dass** die Schnappschließvorrichtung (6) sowohl strukturell als auch funktionell gegenüber einer Achse (2) derart symmetrisch ist, dass sie ohne Unterschied an beiden Flanken (3a, 3b) verwendbar ist.

## Revendications

1. Appareil orthopédique comprenant un cadre métallique (1) qui est en partie rembourré et une ceinture (7) qui en joint un côté (3a) à l'autre côté (3b), comprenant un dispositif de fermeture (6) par encastrement pouvant verrouiller automatiquement à la fin d'un mouvement rotatif avant -arrière ou arrière - avant de ce dernier, le dispositif de fermeture (6) en comprenant un élément fixe (6a) intégralement joint à un côté (3a) et un élément mobile (6c) qui est lié à la ceinture (7) et tourne par rapport à une goupille (6d) à laquelle il est intégralement joint, ladite goupille (6d) coulissant à l'intérieur d'un oeillet (6b) formé sur la surface de l'élément fixe (6a), tandis que l'élément mobile (6c) passe de la position d'ouverture à la position de fermeture, tendant la ceinture (7) et s'engageant au moyen d'une partie rentrante (6e) avec une projection (6f) correspondante de l'élément fixe (6a), **caractérisé en ce que** ladite goupille (6d) coulisse de façon déplaçable dans ledit oeillet (6b).

2. Appareil selon la revendication 1, **caractérisé en ce que** la ceinture (7) supporte des moyens d'association (13) stable mais amovible avec le cadre métallique (1).

3. Appareil selon la revendication 2, **caractérisé en ce que** les moyens d'association (13) consistent en une boucle (13') profilée.

4. Appareil selon la revendication 3, **caractérisé en ce que** la boucle (13') comprend un oeillet (13a) profilé complémentairement à une goupille (9) du cadre métallique (1).

5. Appareil selon la revendication 4, **caractérisé en ce que** la boucle (13') comprend une prise (13b) capable de tendre la ceinture de façon à engager et dégager l'oeillet (13a) et la goupille (9).

6. Appareil selon la revendication 3, **caractérisé en ce que** la boucle (13') comprend au moins un orifice (13c) pour modifier la tension de la ceinture (7).

7. Appareil selon l'une des revendications précédentes, **caractérisé en ce que** le dispositif de fermeture (6) est symétrique à la fois structurellement et fonctionnellement par rapport à un axe (2) de telle sorte qu'il puisse être utilisé également des deux côtés (3a, 3b).
